# EUROPEAN PATENT APPLICATION

(11) **EP 1 334 727 A2**
(43) Date of publication of application: **13.08.2003**
(21) Application number: 01983427.4
(22) Date of filing: 01.11.2001
(51) Int. Cl.: A61K 31/74, A61K 31/785, A61K 39/12, A61K 39/39, A61K 47/30, A61K 47/48, C07K 14/00, C08G 73/02

(54) **METHOD FOR OBTAINING ANTIGENIC STRUCTURES ENHANCING SPECIFIC CROSS REACTIVITY**

(30) Priority: 03.11.2000 CU 2422000
(71) Applicant: CENTRO DE INGENIERIA GENETICA Y BIOTECNOLOGIA (CIGB), Ciudad de la Habana 10600 (CU)
(72) Inventor: CRUZ RICONDO, Luis, Javier;, Padron, 11000 Ciudad Habana (CU); AGUILAR RUBIDO, Julio, César, 32200 La Habana (CU); IGLESIAS PEREZ, Enrique, 11700 Ciudad de la Habana (CU); REYES ACOSTA, Osvaldo, 10600 Ciudad de la Habana (CU); GARAY P REZ, Hilda, Elisa, 10600 Ciudad de la Habana (CU); MUZIO GONZ LEZ, Verena, Lucila, 10400 Ciudad de la Habana (CU); GUILL N NIETO, Gerardo Enrique, 104000 Ciudad de la Habana (CU); DUARTE CANO, Carlos, 10600 Ciudad de la Habana (CU); PENT N ARIAS, Eduardo, 10600 Ciudad de la Habana (CU)
(74) Representative: Prins, Adrianus Willem
(86) International application number: CU0100007
(87) International publication number: WO02036160

(57) **Abstract**

The present invention is related to a method for obtaining antigenic structures with high cross reactivity, able to enhance the immune response to peptidic antigens administered systemically and/or mucosally, as well as with the chemical structures obtained from this method, and with formulations obtained and their uses.

The method described in the present invention enables the obtaining of new antigenic structures, starting from the selection and synthesis of dendrimers, which are coupled to carrier molecules. The resulting structures can be used adjuvated conveniently or in a formulation of various antigens. A synergistic effect can be found in the cross reactivity and immunogenicity. Furthermore, the formulations may contain stabilizers and preserves.

The antigenic structures of the present invention can be used in the pharmaceutical industry as vaccine formulations to prevent or to treat diseases in humans and animals, as well as in the development of diagnostic systems.

## Description

### Technical Sector

The present invention is related with the branch of the medicine, particularly with vaccinate formulations based in new antigenic structures that potentiate cross reactivity.

### Prior Art

The peptides are poorly immunogenicity so that they must be coupled to carrier proteins that provide the required T cell help to elicit specific antibodies (Herrington, D.A. et al. 1987 Nature 328:257-259). During the process of peptide conjugation to carrier protein, the antigenicity properties can be losing due to the inspecifity conjugation. The peptides could be coupled to carrier protein on the main part of the epitope of interest. To avoid the problems associated to the peptide's conjugation, another way of peptide presenting to the immune system has been described. For examples, the Multi-Antigen Peptide (MAP), which is based on incorporation of B and T cells epitope inside a ramified core of lysine (Tam, J. P. et. al. 1988 Proc. Natl. Acad. Sci. 86:5409-54132-5) (Tam, J. P. 1989 Methods Enzymol. 168:7-15) (Tam, J. P. 1990 WO 90/11778) (Tam, J. P. 1993 WO 93/03766). The advantages main of this system are their structure and defined composition as well as the high proportion of the epitope of interest regarding to the total molecule. In some studies immunology, the MAP has been better than the peptide's conjugation (Wang, C. Y. et. al. 1991 Science 254: 285-288). Although this has not always been consistent because in other studies the opposite has happened (Riley, E.M. et. al. 1996 Veterinary Immunology and Immunopathology 55: 243-253), which could be a phenomenon associated to the carrier protein.

The crossed reactivity and neutralization has been described previously for peptide synthetic (Wang, C. Y. et. al. 1991 Science 254: 285-288). Several investigators have also reported that anti-peptide antibodies against the V3 region of the HIV-1 present crossed neutralization toward different isolations of field (Wang, C. Y. et. al. 1991 Science 254: 285-288). In summary, the crossed neutralization and reactivity is associated to the peptidic structure. For instance, peptides of the V3 loop of HIV-1.

The MAP strategy has been used to generate a neutrality response anti V3 in human (Geoffrey, J. et. al. 1996 Journal of Infectious Diseases, 173: 330-3398). The disadvantage of this methodology consisted in that for obtaining an effect the neutralization was necessary to inoculate an excessively high quantity, 500 µg, of MAP. (Kahn, J. et. al. 1994, Tenth International Conference on AIDS Yokohama, Japan 7-12 August., Vol. 1.) (Li, D. et al. 1997 seized Pac J Allergy Immunol. 15:105-13). It was found that a mixture of MAPs based on the selection of V3 different, looking for the widest spectrum of neutralization of clinical isolations, the necessary quantity of total micrograms of MAP it would be incompatible with the habitual practices of immunization.

The crossed reactivity is defined as the ability that possesses an immunogen to react with the related ligands (Paul, Fundamental W. Immunology. 4th edition). Although the phenomenon of the crossreactivity is known from the beginnings of the immunology, up to now, any vaccine preparation has been based on the cross reactivity. This, maybe, explains why the immense majority of the vaccines used in the programs of immunization do not protect against pathogens with high variability among their several clinical isolates.

The crossed reactivity among homology peptides is a property of each sequence. It can be enhance according to the forms of presentation of the peptide to the immune system. It's not obvious that the enhancing of the crossed reactivity is given by an increase of the immunogenicity. In a study with T-B peptide was demonstrated that the induction of antibodies with crossed reactivity and/or neutralization, don't correlate with the peptide immunogenicity (Kasmi, K.C, et. al. 1998 molecular Immunology 35:905-918).

Different strategies have been generated for obtaining a immune response able to recognize a great number of antigenic variant of a pathogen. One of these consists, in the design of Multi-Epitope Polypeptide recombinant (MEPr) for the variants more important. The presentation of B epitope in the MEPr system not always has been a viable option (Flynn, J.N, et. al. 1997 J Virol 71: 7586-92). In first term, the generation of a chimeric polypeptide implies the production of a molecule of unpredictable three-dimensional structure that does not have to expose all the epitopes appropriately. This design favors the response toward some epitope or impedes the response totally against others. On the other hand, it is also unpredictable the dominance that will be imposed among the different epitopes. Another strategy has been the generation of peptide's libraries as immunogen. The peptide's library was obtained of highly variable regions. It represents a great number peptide sequence. However, it has the difficulty of the little representativeness of each epitope. It was diluted inside a very big heap of different sequences, which makes unpredictable an immune response at the required level (Allen, D. et. al. 1998 J. of Virol. 27: 651-659)(Jerome, E. et. al. 1994 Eur. J. Immunol. 24: 2789-2795).

Another strategy is the generation of consent peptide with the objective of covering a wide spectrum of sequences V3, in the case of the HIV. This is based on the study of the epitope variability in the field isolations or clinical, settling down for each position to the amino acid more conserved among all the isolations (Holley, L.H. et. al. 1991 Proc. Natl. Acad. Sci. 88:6800-6804).

### Disclosure of the invention

The technical objective of the proposed invention is the development of a method for obtaining antigenic structures of high crossed reactivity to able enhances the immunogenicity of peptidic antigens, administered by systemic and/or mucosal route. This method generates dendrimer structures conjugated to carrier proteins in appropriate proportions. The addition of similar structures carrying other B epitope has allowed increasing the crossed reactivity. Besides, it is also gotten an increment of the immunogenicity of the antigens co-administered. Other antigens and adjuvants can be added to this preparation.

Therefore, one of the objects of the present invention is a method to synthesize antigenic structures that enhance the specific cross reactivity.

The above mentioned method comprises the following steps:
A) Selection of epitopes of interest;
B) Obtaining of dendrimeric structures having selected epitopes of step (A), using a method of synthesis, which can be the synthesis on a lysine or aspartic acid cores; synthesis on organic molecule cores; synthesis on a dendrimeric core; peptides coupling on a dendrimeric core; or mixtures on a single core of B-cell epitopes, T-cell epitopes or both.
(C) Conjugation of one or several dendrimeric structures obtained in step (B), to one or several carrier molecules using a conjugation method which can be with glutaraldehyde, succinic anhydride, ester of the β-maleimidopropionic-N-hidroxisuccinimic acid (MPS) or with a spacer agent without aromatic side chains, because it was shown that such groups avoid the recognition of coupled dendrimeric structures.
D) Mixture of conjugates obtained in step (C) for obtaining formulation by adding of the CD4 binding peptide or a conjugate having that epitope, which enhance the cross reactivity and immunogenicity of the resulting antigenic structure; and finally
E) To adjuvate in alum or any proper adjuvant.

In the method of the present invention, peptides in structures obtained in step (B) can be regions from HIV 1 or HIV 2, more particularly variable regions like the V3 loop.

Also regions having some variability from other viruses among them, the HCV, HBV and Dengue Viruses can be used.

Other antigens from polysaccharidic regions from Neisseria meningitidis, or mimetic peptides from polysaccharidic antigens, or any other region from a microorganism or pathogen can be used.

Additionally, the method of the present invention can use regions from self antigens or relevant antigens for a cancer vaccine, for instance, regions from the Gonadotropine Releasing Hormone.

Among carrier proteins that can be used in the method of the present invention are the surface antigen from the Hepatitis B Virus, (HBsAg), the p64K protein from Neisseria meningitidis, the core antigen from the HIV, the core antigen from the Hepatitis B. Also, polysaccharidic molecules like dextrans can be used for the same purpose.

The method of the present invention comprises the conjugation of one or several dendrimeric structures obtained in step (B), to one or several carrier molecules by covalent conjugation, or by electrostatic or hydrophobic interaction.

Another object of the present invention is the antigenic structure obtained by the former method, which enhances the specific cross reactivity of the epitopes contained. It comprises epitopes previously described as dendrimeric antigens coupled to carrier proteins being 1-30% of the resulting structure.

The selected epitopes can be of polysaccharidic or peptidic nature. Peptides can be consensus peptides from variable regions selected by subtypes, subgroups, serotypes or any classification. In addition, they can be peptide libraries or mimetic peptides from a microorganism.

In particular, peptides in dendrimers can be variable regions from HIV 1 or HIV 2, among the V3 loop; and regions from other viruses with some variability and diversity like Hepatitis B, Hepatitis C and Dengue Viruses.

These structures comprise antigens of polysaccharidic regions from Neisseria meningitidis, mimetic peptides for polysaccharidic antigens or any regions from a microorganism or pathogen.

Additionally, the antigenic structure of the present invention can use regions from self antigens or relevant antigens for a cancer vaccine, for instance, regions from the Gonadotropine Releasing Hormone. Among carrier proteins, which can be used by the method of the present invention, are the surface antigen from the Hepatitis B Virus, (HBsAg), the p64K protein from Neisseria meningitidis, the core antigen from the HIV, the core antigen from the Hepatitis B. Also, polysaccharidic molecules like dextrans can be used for the same purpose.

The invention is also related with the use of antigenic structures already described for diagnostic or for prophylactic or therapeutic vaccine formulations for infectious, autoimmune or cancerous diseases, which can be inoculated by systemic routes.

In particular, for the V3 loop it was demonstrated that it is possible to increase much more the cross reaction and cross neutralization by adding conjugates having the CD4 binding peptide on the same structure developed to enhance cross reacting antibodies against peptides from variable regions.

The addition of such structures increases the anti-V3 cross reaction and immunogenicity of coadministered antigens. In fact, the method make easier the appearance of cross reacting antibodies in vaccinal strategies based on them, but it is not excluded its use in vaccinal strategies just to increase immunogenicity to the homologous peptide.

The invention provides a methodology for obtaining new chemical structures based on a differential presentation of B-cell epitopes from variable microorganisms to the immune system. Such variability could be related to the pathogenicity. In addition, non-variable B-cell epitopes capable to elicit a protective immune response, likewise B-cell epitopes from cell antigens can be used. All of them in dendrimeric structures on carrier proteins capable to enhance their cross reaction and immunogenicity.

The proposed method allows producing vaccinal antigens in a non-conventional way having a higher epitopic density, which increases immunogenicity ant anti-peptide cross reactivity. Also, the method allows to decrease amounts of dendrimeric structures eliciting higher antibody titers appart from the new features already described because of the differential presentation.

It is important to point out that for peptides from variable regions is possible to increase the cross reactivity without any effect on their immunogenicity as described in

### Examples.

On the other side, this method allows to couple different dendrimeric structures to the same carrier. Likewise, it was demonstrated that mixtures of structures of the present invention with a similar having the CD4-biding peptide increases the anti V3 immunogenicity and cross reactivity.

The new structure can comprise a dendrimeric portion having only B-cell epitopes because the carrier provides T-cell epitopes. MAP structures used as vaccine elements avoids the use of a carrier and the T-cell epitopes of the present structures can be included in the synthetic portion or in the carrier, optionally. This strategy brings a better presentation for B-cell epitopes because they are not in compact clusters. Also, using dendrimers coupled to carrier proteins it is possible to enhance cross reaction and/or immunogenicity using less quantity of immunogen in comparison to dendrimers alone (ex. MAPs).

Finally, it was shown a synergistic effect over immunogenicity and cross reaction in mixtures of those conjugates.

The formulation of the present invention can be inoculated through the mucosal route, enabling to this technology to reach their use by this route, a very important one nowadays.

### EXAMPLES

### Example 1: Method for obtaining the antigenic structures.

In general, the syntheses of peptides, MAPs and dendrimers have been described previously (Tam, J. P. et. al. 1988 Proc. Natl. Acad. Sci. 86:5409-5413) (Shao J. et. al.

1995 J. Am. Chem. Soc. 117:3893-3899). The antigenic structures are obtained by cross linking of the dendrimer structures to carrier proteins. It was carried out using the succinic anhydride (SA) as spacer agent. It permit to link covalently to both antigens through its amino groups. For this, the carrier protein was activated with succinic anhydride in buffer tampon pH 8.5 during 30 minutes. Then, the reactive remains are eliminated by dialysis to avoid the cross linker between dendrimer molecules (MAP) and peptidic not wanted. The conjugation was carried out by activating of the carboxyl groups of protein with ECDI (soluble carbodiimide). Subsequently, the synthetic antigen was added. The separation of those conjugated was performed by filtration in gel. Finally, they were characterized by ELISA and Western Blotting using AcM and serums of mice.

### Example 2: Method for obtaining the antigenic structures using different spacer agents.

The obtaining of the antigenic structures was carried out using different spacer agents. A desirable requirement for obtaining a conjugated of good immunologic quality is that the union to the protein is carried out far of the residuals important of the epitope. To avoid this phenomenon, different spacer agents have been used: glutaraldehyde, m-maleimidobenzoyl-N-hydroxysuccinimide ester (MBS), carbodiimides, β-maleimidopropionic acid N-hydroxysuccinimide ester (MPS), succinic anhydride and others. During immunization, antibodies against the carrier proteins and/or the coupling agent might be elicited. Ideally, a good coupling agent should not generate any antibodies response against itself.

It was found that that the antibody response toward V3-based synthetic multiple antigen peptides (MAP) derived from HIV-1, JY1 isolate, conjugated to two different carrier proteins using either m-maleimidobenzoyl-N-hydroxysuccinimide ester (MBS), or β-maleimidopropionic acid N-hydroxysuccinimide ester (MPS), or succinic anhydride show different behavior. An excellent anti-JY1 response without a strong response to the coupling agent is observed in the case of succinic anhydride spacer. In contrast, MBS produces total abrogation of the antibody response with a high response toward the coupling agent. This result demonstrates that not all the spacer agents reported in the literature present in same behavior, which indicates that in the obtaining of this type of antigenic structures it is not obvious the use of the spacer agents previously described in the state of the art.

### Example 3: Design, synthesis and conjugation of new dendrimeric structures to the HBsAg.

Considering that, former antigenic structures (i. e., MAP-protein conjugates) elicited high titers against interesting peptides new dendrimeric structures were synthesized. This time without Th epitope because the carrier protein provide them. Because of that the synthesis of such structures become easier, longer B cell epitopes would be included and un-wanted 3D structures are avoid because of the co-linearity between B and Th epitopes. Dendrimers were coupled individually to HBsAg as described in example 1. MAPs were synthesized as referred by J. Tam et. al. (1988). Afterwards, dendrimeric structures and conjugates were separated by gel filtration chromatography and the concentration was determined by the Comassie method.

### Example 4: Study of immuno-potentiation and enhance of the cross reactivity.

With the aim to study the effect of MAP conjugation to a carrier protein on the immunogenicity and cross-reaction two groups of 10 female mice Balb/c 6-8 week olds were immunized using subcutaneous route in FCA/FIA. Groups were: 1, MAP8 (TT-JY1) and 2, HBsAg-MAP8 (TT-JY1). Doses of 50 y 10 µg were inoculated, respectively. Mice were immunized on days 0-14-28-40 and they were bled on days 36 and 51. The HBsAg-MAP8 (TT-JY1) was obtained using succinic anhydride as spacer as described in example 1. The MAP8 (TT-JY1) has eight copies of the T cell epitope, amino acids 830-844 from the tetanus toxin (TT), and eight copies of the B cell epitope, comprising the central 15 amino acids from the V3 region of gp120 of HIV-1 JY1 isolate (Cruz, L.J. et al. 2000 Journal of Peptide Science 6: 217-224). After third and fourth doses antibody responses against the JY1 epitope and other V3 peptides were study. The IgG response in sera was quantified by indirect ELISA, using peptides coupled to BSA (Gómez, C.E. et al. 1998 J. Virol. Methods. 71: 7-1618). Pooled sera were used 100⁻¹ diluted.

Statistical analyses were done by the Student's test. Probability values less than 0.05 were considered statistically significant and less than 0.01 highly significant.

After the third dose no difference was found regarding immunogenicity to the homologous peptide thought five times less mass of the conjugate was inoculated.

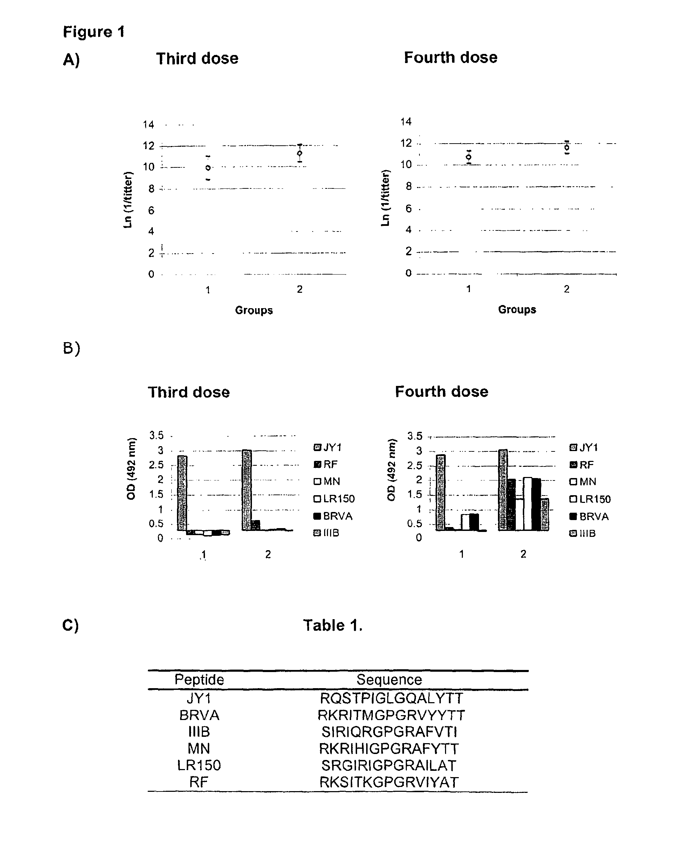

Therefore, using conjugates a big economy of antigen is possible which is an important advantage (Fig. 1A). In the group immunized with HBsAg-MAP8 (JY1-TT) cross-reaction to other V3 peptides appeared in contrast to mice inoculated with MAP8 (TT-JY1), which were not cross-reactive, thought similar levels of antibody responses were achieved. After four doses, the HBsAg MAP8 (TT-JY1) induced a higher response to the JY1 epitope compared to MAP8 (TT-JY1). Some cross-reacting antibodies were elicited in the MAP8 (TT-JY1) group, which were limited in comparison to the HBsAg-MAP8 (JY1-TT). The increase of immunogenicity is very important since it has been reported the use of MAP cocktails to elicited antibodies against several epitopes. However, the total mass needed for obtaining the wanted effect (i. e., neutralization, etcetera) might be in some cases no allowed to be inoculated to human beings because of possible side effects. As a conclusion, it is very important to induce a high antibody response with limited mass of Antigen. Therefore, it is evident the advantage of such structures (i. e., MAP-carrier protein conjugate) described in this methodology. Taking into consideration that using less amount of antigen, a broaden cross-reactive response can be elicited, the quantity of variable epitopes to inoculate in a cocktail would be decreased consequently. Finally, the use of such conjugates would be an approach to produced bivalent vaccines when the carrier is per se a protective immunogen like the HBsAg.

### Example 5: Study of the humoral response of mice immunized with MAP8 (TT-JY1) and TAB9.

With the aim to study differences related to the presentation of the JY1 epitope to the immune system (I.S.), the recombinant protein TAB9 (Duarte, C.A. et al. 1994 AIDS Res Hum Retroviruses.10: 235-243) it comprises V3 peptides from several HIV-1 isolates; LR150, JY1, RF, MN, BRVA and IIIB in that order. They are 15 amino acid long involving the central part of the V3 loop) and MAP8 (TT-JY1) were compared in a single experiment. It was studied the recognition to V3 peptides included in TAB9 and the differences were assessed by epitope mapping using Ala substituted peptides spanning the whole JY1 sequence. When an Ala was present in the sequence it was substituted by Gly. Two groups of 10 female Balb/c mice 6-8 weeks old were immunized by subcutaneous route in CFA/IFA with 100 µg of TAB9 protein and 16.4 µg of MAP8 (TT-JY1). Mass of both immunogens was set at equal quantity of JY1 epitope.

Mice were immunized on days 0, 14, 28, 56 and bled on day 67. The anti-JY1 IgG response in sera was measured by ELISA.

Statistical analysis was done by the Student's test. Probability values less than 0.05 were considered statistically significant and less than 0.01 highly significant.

To study cross-reaction to V3 peptides IgG response in sera were quantified by indirect ELISA using peptides coupled to BSA (Gómez, C.E. et al. 1998 J. Virol. Methods. 71: 7-1618). Pooled sera were used 100⁻¹ diluted.

The epitope mapping on cellulose membrane has been described before by Ronald Frank (Frank, R. 1992 Tetrahedron 48: 9217-9232).

Pooled sera after four doses from groups were tested against several V3 peptides. It was found that mice immunized with MAP8 (TT-JY1) were able to detect four out five (LR150, RF, MN, BRVA) V3 tested. Among them three (RF, MN, BRVA) gave absorbance values (A_{492 nm}) higher than 1.25 being strong reactivities. For the RF peptide, in particular, the A_{492 nm} was higher than 2.5. The former results illustrate the advantage of MAPs for presentation of B cell epitopes to the I. S. In the case of TAB9 protein comprising the whole peptides, tested two peptides (RF y BRVA) were not recognized. Peptides MN and IIIB were detected at very low A_{492 nm} values, less than 0.75. Only JY1 and LR150 peptides were recognized at very high A_{492 nm} values. We conclude that presentation to the I. S. by MAP makes feasible the cross-reaction to other V3 peptides not included in the immunogen to appear. Because of that, the quantity of antigens to be included in a strategy to deal with a universe of homologous sequences would be optimized. The result for TAB9 protein probed that inclusion of several sequences in a recombinant protein is not always a feasible technical solution.

In fact, the MAP8 (TT-JY1) showed a better response.

Trying to find out differences in the presentation of epitopes to the immune system epitope mapping using overlapping and Ala substituted peptides from the JY1 sequence were carried out. It was found that pooled sera from both groups recognized the same amino acid sequence, LGQALY. For MAP8 (TT-JY1) group amino acid L-Q--Y were directly involved in the interaction with Abs. In contrast, for TAB9 amino acid L-Q-LY were mainly involved. That slight difference of only one amino acid seems to be the cause of differential recognition to V3 tested peptides. Thought, it is possible to speculate that the epitope is displayed in different 3D structures in both immunogens.

In conclusion, peptides in TAB9 protein are differentially exposed to the immune system in comparison to the MAP. This experiment probed that such differential exposition is responsible of cross-reacting profiles in both immunogens.

### Example 6: Immunogenicity comparison among different ways of peptide presentation to the immune system.

With the aim to evaluate the immunogenicity of several ways of peptide presentation to the immune system, nine groups of 8 female Balb/c mice were inoculated. Animals were immunized on days 0-14-28 and bled 10 days after the last immunization. All data are summarized in Table 2. The MAP4 (TT-JY1) has four B cell epitopes (JY1 all of them) and four T cell epitopes (TT₈₃₀₋₈₄₄ all of them). The peptide (TT-JY1) has the B (JY1) and T (TT) cell epitopes in tandem. The linear polymer was synthesized by polymerization from Cys added at both sides of the former peptide (Cruz, L.J. et al. 2000 Biotecnologia Aplicada 17:35-38). Also, the peptide was synthesized on the surface of dextran beads. HBsAg-peptide (TT-JY1), HBsAg-MAP4 (TT-JY1) and HBsAg-MAP8 (TT-JY1) were obtained by conjugation using succinic anhydride (AS) as spacer as already described in example 1. The group HBsAg (group 9) was added as placebo. The anti-JY1 IgG response in sera was measured by ELISA.

Statistical analyses were done by the Student's test. Probability values less than 0.05 were considered statistically significant and less than 0.01 highly significant.

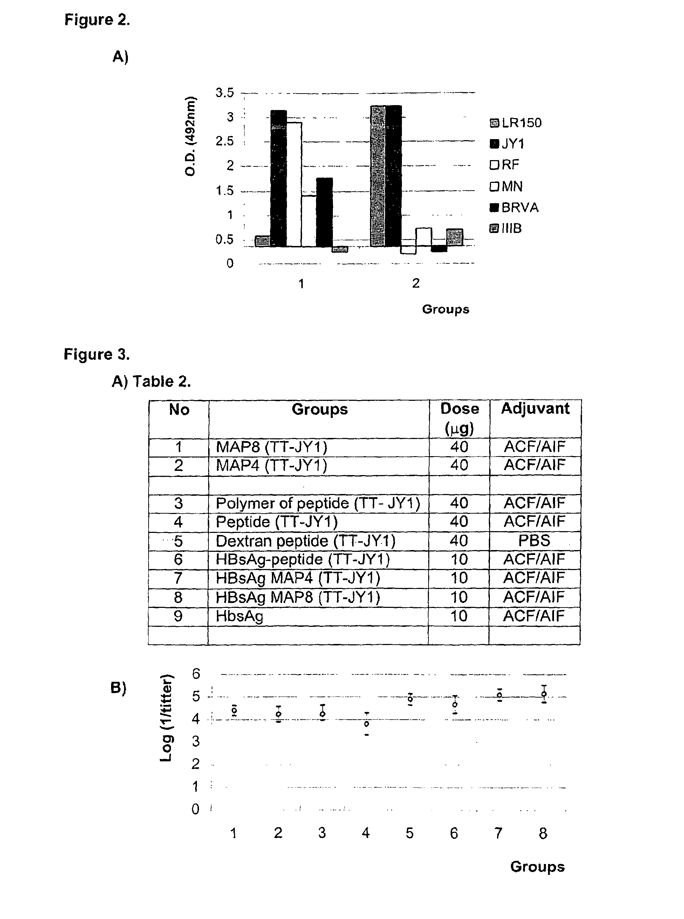

It was probed that MAP-HBsAg conjugates (groups 7 and 8) elicited significant higher antibody response against the JY1 epitope in comparison to MAPs and the polymer. The former groups were significantly superiors than the peptide (group 4). The dextran-peptide (group 5) (Fig 3B) did not differ from HBsAg-MAP conjugates. However, it should be considered that four times less mass of conjugates was inoculated. This experiment probed that conjugation of MAPs to HBsAg enhanced significantly the antibody response against the JY1 epitope in comparison to MAPs. Thought MAPs were developed to avoid peptide conjugation to carrier proteins we have shown that after conjugation the immune response is enhanced over the antibody response elicited by four times more mass of them inoculated alone. It is concluded that MAP-carrier protein (i. e., HBsAg) conjugates are the best of tested immunogens.

### Example 7: Study of cross-reactivity elicited by different presentation ways of synthetic peptides to the immune system

With the aim to evaluate the level of cross-reaction elicited by different presentation ways of the JY1 peptide Pooled sera corresponding to different immunization schedules were set at approximately 10⁻⁵ anti-JY1 titer and compared regarding their capacity to cross-react with a panel of five V3 peptides from different HIV-1 isolates. In all cases groups of 10 female Balb/c mice were inoculated using subcutaneous route with a volume of 100 µL. The general schedule of immunization was on days 0, 14, 28, 56, and 99. Animals were bled 10 days after the final dose. All groups were not inoculated five times. Data are summarized in Table 3. Groups having the same schedule number were extracted from the same experiment.

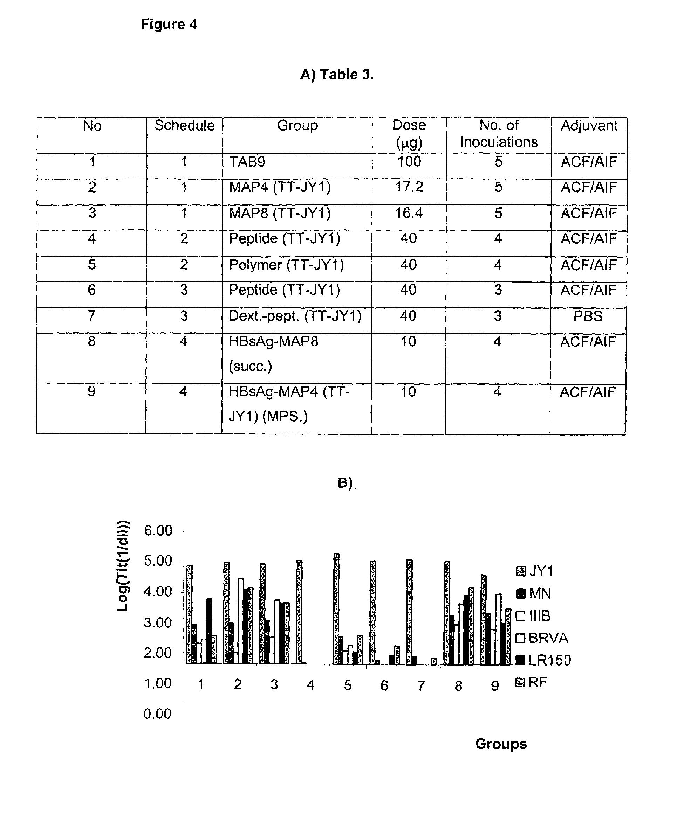

Results of this experiment evidence that cross-reaction is a feature of the JY1 V3 peptide per se because it is present in every way of presentation to the immune system tested (groups 2, 3, 8 and 9). In addition, such cross-reaction could be higher than the response elicited by an immunogen comprising all tested peptides (groups 1, 2, 3, 8 and 9) (Fig. 4B). Regarding cross-reaction MAP4 (TT-JY1), MAP8 (TT-JY1), HBsAg-MAP4 (TT-JY1) and HBsAg- MAP8 (TT-JY1) were not different. However, it is important to observe that mice immunized with conjugates received a lesser quantity of protein having just four doses instead of five like groups inoculated with MAPs. The cross-reaction analysis for MAPs after four doses (example 4) showed a lower level than conjugates, which evidences the enhancing effect after the conjugation. So, this experiment supports results in example 4.

### Example 8: Immunological evaluation of new dendrimer-HBsAg conjugates. Sera recognition against a panel of V3 peptides.

With the aim to evaluate possible interactions among different conjugates in a vaccinal formulation and with the aim to demonstrate the enhancing activity on the immunogenicity and cross-reactivity of mixtures of conjugates an experiment in mice was carried out.

Ten groups of 10 female Balb/c mice were inoculated using subcutaneous rote, in a volume of 100 µL. Immunization groups were distributed as follows: 1, HBsAg-MAP4 (consensus peptide from V3); 2, HBsAg-MAP4 (CD4 binding peptide from gp120); 3, Mixture of conjugates HBsAg-MAP4 (consensus peptide from V3) and HBsAg-MAP4 (CD4 binding peptide from gp120); 4, HBsAg-MAP4 (peptide library of V3 sequences); 5, Mixture of conjugates HBsAg-MAP4 (peptide library of V3 sequences) y HBsAg-MAP4 (CD4 binding peptide from gp120). Peptide sequences are summarized in Table 5. Doses of 10 µg were inoculated in groups 1, 2 and 4; for groups 3 and 5 only 5 µg every conjugate. Mice were immunized on days 0, 14, 28, 56 and 84. They were bled 10 days after the last immunization. Data are summarized in Table 4.

Cross-reacting Abs were study against a panel of 50 V3 peptides from different HIV-1 isolates synthesized on cellulose membrane. Also, cross-reacting Abs were evaluated in ELISA against five peptides already tested in the example of figure 1. Pooled sera from every group were used 1/100 diluted.

Antibody responses in sera were measured by ELISA determining anti IgG response to consensus peptide, CD4 binding peptide from gp120 and peptide library of V3 sequences.

Statistical analyses were done by the Student's test. Probability values less than 0.05 were considered statistically significant and less than 0.01 highly significant.

After five doses, the antibody responses against the consensus peptide in groups HBsAg-MAP4 (peptide library of V3 sequences) and HBsAg-MAP4 (consensus peptide from V3) were very weak (Table 6). It is interesting to note that pooled sera from animals immunized with HBsAg-MAP4 (peptide library of V3 sequences) did not react with any of V3 peptides tested, neither with the MAP4 (consensus peptide from V3) not with the consensus peptide coupled to BSA. However, in the group inoculated with a mixture of HBsAg-MAP4 (CD4 binding peptide from gp120) and HBsAg-MAP4 (peptide library of V3 sequences) a high antibody response against the consensus peptide and MAP4 (peptide library of V3 sequences) appeared. Such responses were not consequence of anti-CD4 binding peptide Abs because sera from mice immunized with HBsAg-MAP4 (CD4 binding peptide from gp120) did not react with any of those peptides. So, it is concluded that because a synergic effect in that mixture of conjugates antibody responses against consensus peptide and V3 library were enhanced. Then the MAP4 (CD4 binding peptide from gp120) had an immuno enhancing effect on the anti-V3 response which is very strong using twice less immunogen mass in the mixture in comparison to conjugates inoculated alone. The same synergic effect is present in the group HBsAg-MAP4 (consensus peptide from V3) and HBsAg-MAP4 (CD4 binding peptide from gp120).

This experiment probed that mice immunized with mixtures of conjugates (groups 3 and 5) developed Abs against B cell epitopes included in the formulation (Table 6). These conjugates were prepared individually and then mixed to avoid differences in the respective proportions. Because of that three different mixtures of conjugates developed similar levels of anti-peptide antibody responses.

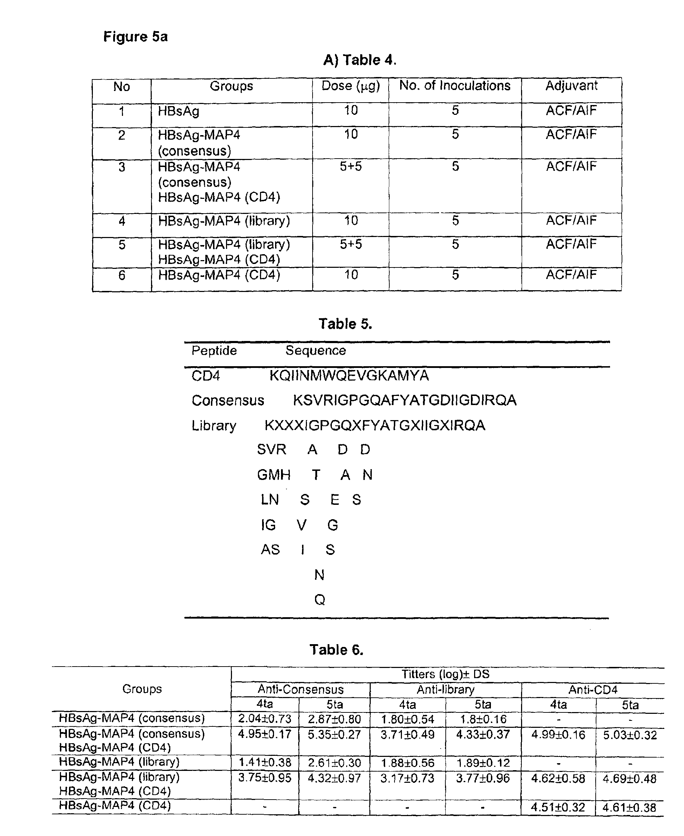

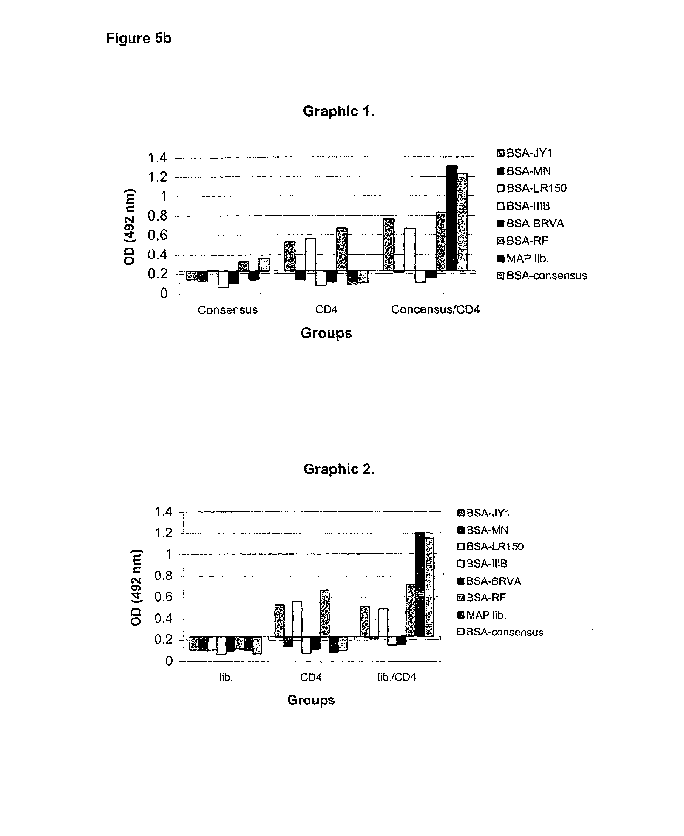

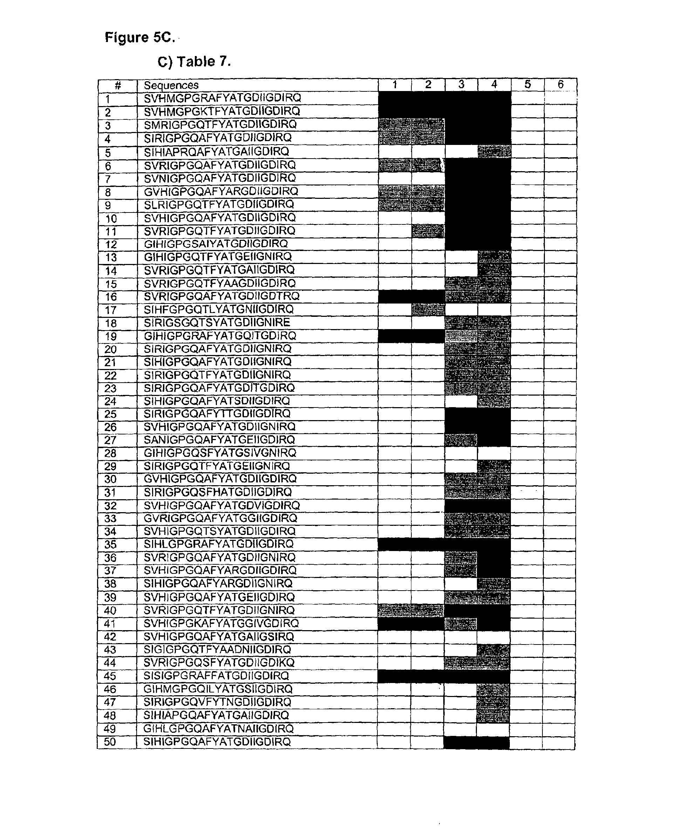

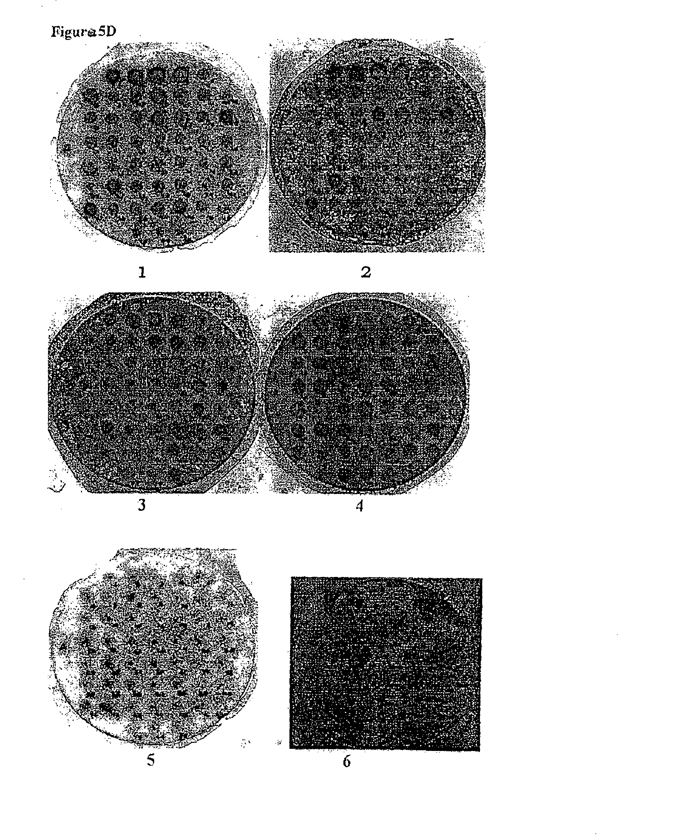

Anti-consensus peptide and anti-peptide library antibody responses were significantly higher when HBsAg-MAP4 (consensus peptide from V3) and HBsAg-MAP4 (peptide library of V3 sequences) were inoculated in a mixture with the CD4 binding peptide from gp120 in comparison to them immunized alone. The anti-consensus response increased and the anti-peptide library appeared to an important level as it is presented in figure 5b (graphics 1 and 2). Nevertheless, in the mixture half of the dose for the individual conjugates was inoculated. This result evidenced that it is possible to enhance the anti-V3 response in formulations having the MAP4 (CD4 binding peptide from gp120 (Figure 5, graphics 1 and 2).

The increase in cross-reactivity was evidenced by anti-V3 peptide ELISAs. Sera from this experiment were tested against the panel V3 peptides in TAB9 protein already described in figure 1. Regarding the interaction between consensus and CD4 peptides it was observed that the mixture elicited high titers because of cross-reacting Abs elicited by the CD4 peptide and the normal reactivity of the consensus peptide increased by the enhancing effect of the HBsAg-MAP4 (CD4 binding peptide from gp120). Additionally, pooled sera from groups 3 and 5 were analyzed after four and five doses against a panel of 50 V3 peptides. In the case of group 5 (mixture of conjugates HBsAg-MAP4 (peptide library of V3 sequences) and HBsAg-MAP4 (CD4 binding peptide from gp120)) after four doses 26% (13/50) of tested peptide were recognized.

The percentage comes to 30% (15/50) after five doses. After four doses 53% (7/13) recognized peptides had a very strong reactivity and after five doses 46% (7/15) of them.

Mixture in group 3 (HBsAg-MAP4 (consensus peptide from V3) and HBsAg-MAP4 (CD4 binding peptide from gp120) after four doses reacted with 72% (36/50) of peptides and a 92% (46/50) of them after five doses. After four doses 50% (18/36) recognized peptides had a very strong reactivity and after five doses 48% (22/46) of them.

It was concluded that the mixture HBsAg-MAP4 (consensus peptide from V3) and HBsAg-MAP4 (CD4 binding peptide from gp120) elicited a broaden cross-reacting response than a similar mixture with the peptide library. Since the consensus peptide was created from the selection of more frequent amino acids in every position in the panel of 50 peptides and the library was generated from the same panel, we conclude that the best strategy to elicite cross-reacting antibodies, is a formulation comprising a mixture of MAP of a consensus peptide coupled to a carrier protein plus another conjugate having a synergic effect.

### Example 9: Comparison of the immune response generated by the linear V3 (JY1) peptide and the MAP coupled to different carrier proteins.

To study the effect of conjugating dendrimeric structures (in this case MAPs) to different carrier proteins on the immunogenicity and crossreactivity, the linear JY1 peptide and its MAP structure were coupled to different carrier proteins (HBsAg, KLH, P64k).

The method of succinic anhydride was used to couple the peptide and the MAP to the different carrier proteins used in the groups 5 to 9. The MAP of group 10 was coupled to the HBsAg using MPS as a coupling agent.

Nine groups of 8 Balb/c female mice were inoculated on days 0-14-28-56 and the extraction was carried out 10 days after the last inoculation. Immunization groups, doses and adjuvants used are presented on table 8.

The antibody response was quantified by ELISA for IgG against JY1 peptide in serum. The cross reactivity was also evaluated by ELISA employing a panel of peptides as described in example 5, furthermore, a non-related peptide was used as control.

The test F to analyze the variance homogeneity followed by the student t test, were used to analyze the results. Values of p<0.05 were considered statistically different and p<0.01 highly significant.

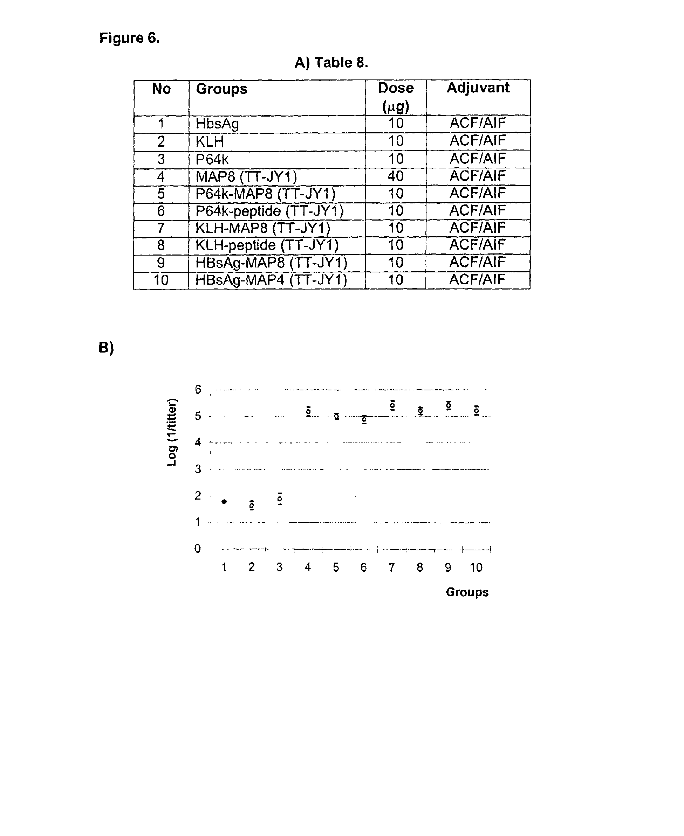

The antibody response to the conjugate protein-MAPs was in all cases superior to the response generated by the conjugate peptide-proteins (fig. 6B). The response against HBsAg-MAP8 (TT-JY1) was similar to the response of KLH-MAP8 (TT-JY1) and both were significantly superior to the response obtained with the conjugate P64k-MAP8 (TT-JY1). In the other hand, the antibody responses against the conjugates of MAPs were also significantly superior (p<0.05) to the one generated by MAPs alone. Except for the case of P64k-MAP8 (TT-JY1) although is very important to point out that the total amount of antigen in this conjugate is lower. These results demonstrate that the use of dendrimeric structures coupled to carrier proteins optimizes the total amount of immunogen to use and is possible besides to enhance the immune response significantly.

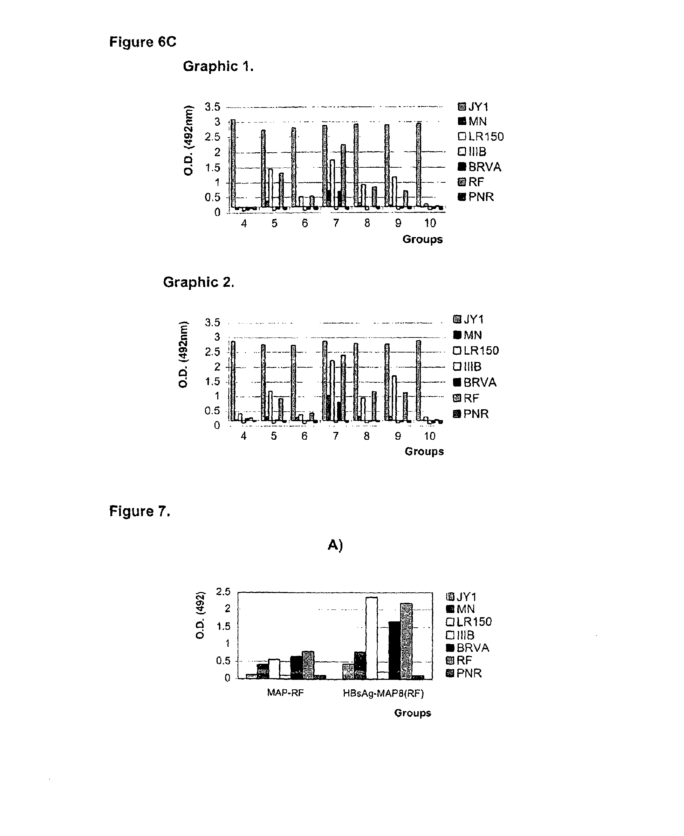

The cross reactivity was observed immunizing with all the conjugates versus more than two heterologous V3 peptides at least and was growing up after third and fourth doses (fig. 6C). Furthermore, it was possible to evidence that the cross reactivity was significantly superior for coupled MAPs compared to uncoupled ones (fig. 6C).

### Example 10: Determination of immunogenicity and cross reactivity in sera after immunization using a different V3 isolate on dendrimeric structures.

With the aim of demonstrating that the enhancing effect found on the immunogenicity and cross reactivity does not depend on the selected V3 sequence, both, immunogenicity and cross reactivity were measured after immunizing with dendrimer5ic structures of a different V3 peptide corresponding to the V3 isolate. The sequence of RF peptide has been described on table 1, figure 1.

Eight to ten weeks old female Balb/C mice were inoculated on days 0, 14 and 28, the extractions were pre immune and after the third dose. The inoculated antigens were the MAP8 (TT-RF), and the dendrimeric structure obtained by coupling the RF-based MAP to the HBsAg employing the coupling agent succinic anhidride as previously described in the example 1. Doses employed were 40 and 10 µg respectively.

As it is clearly observed in figure 7, both, the dendrimeric structure coupled to the HBsAg as well as the MAP generated significant cross reaction levels and after the statistical analysis it was demonstrated the significant increase on the immunogenicity and cross reactivity for sera from the group immunized with the dendrimeric conjugate HBsAg-MAP8 (TT-RF).

### Example 11: Immunological analysis of different MAPs conjugated to carrier proteins.

With the objective of studying the effect of potentiation on the immunogenicity of different MAPs covalently joined to carrier proteins, tetrameric MAPs were synthesized and the corresponding peptide in their lineal shape. The MAP4-(NM) and the MN peptide contains a derived epitope from the loop 4 derived of the OMP P1.15, strain

B385 of Neisseria meningitidis (HYTRQNNADVFVPAWG). The MAP4-(D) and the D peptide containing an epitope derived from the Dengue 2 virus preM/M protein (LTTRNGEPHMIVSRQEKGKSLL FKTGDGVG).

The MAP4-(NM) and the MN peptide were conjugated to the carrier proteins KLH and P64k employing as coupling agent the ester of the β-maleimidopropionic-N-hidroxisuccinimide acid (MPS). The reaction of conjugation was carried out by derivatization of the proteins with an excess of spacing agent in phosphate buffer 50 mmol/L in DMF at pH 6 during 30 minutes. The excess of MPS was eliminated by dialysis. During the synthesis of MAP4-(NM) and the MN peptide a cystein was added in the C-terminal generating a group -SH. Derivatized proteins with the maleimide reacted spontaneously with free tiol groups on respective peptides and MAPs during 3 h at room temperature. The purification of these conjugates was carried out by gel filtration chromatography.

In the case of MAP4-(D) and the D peptide were conjugated to the proteins KLH and P64k using the direct method of soluble carbodiimide. The MAP4-(D) and the the D peptide contains a carboxyl group in the C-terminal, enabling the direct binding to the amine groups from the carrier proteins (KLH and P64k).

To evaluate the immunogenicity of the different conjugated MAPs, twelve groups of 8 female Balb/c mice were immunized using subcutaneous route. The inoculations were on days 0-14-28. Mice were bled 10 days after the last inoculation. Immunized groups, doses and adjuvant used are presented in table 8.

The antibody response was quantified by ELISA, to determine total IgG against the corresponding peptide in sera.

The test F to analyze the variance homogeneity followed by the student t test, were used to analyze the results. Values of p<0.05 were considered statistically different and p<0.01 highly significant.

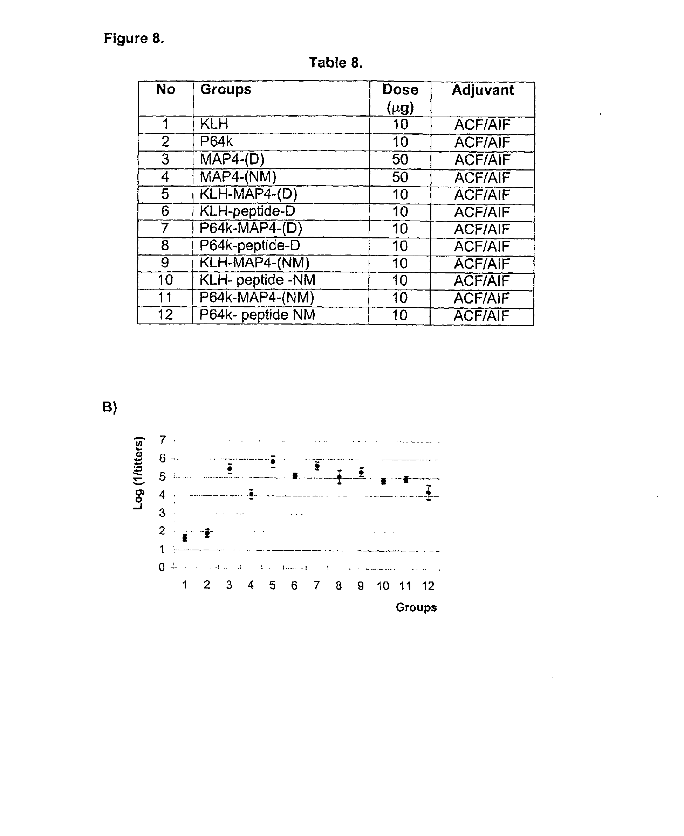

As it is observed in figure 8B, higher titers corresponded to the conjugates KLH-MAP4-(D) and P64k-MAP4-(D). In all cases, the titers of conjugates protein-MAPs were significantly higher to the conjugates protein-peptides, which achieved responses of similar intensity of groups immunized with MAPs alone. From this experiment we concluded that the conjugation of dendrimeric structures containing sequences of different pathogens to carrier proteins make possible a significant increase on the resulting immunogenicity towards the selected peptide.

### Example 12: Study of cross reactivity after nasal immunization using dendrimeric conjugates.

With the aim of demonstrating the generation of antibody responses with high cross reactivity after nasal immunization, groups of 8 female Balb/c mice from 10 to 12 weeks old were inoculated with 10 µg of HBsAg coupled to the (TT-JY1) MAP8. As a control we used groups of 8 mice administered with 40 µg of (TT-JY1) MAP8, the antibody response against both immunogens was evaluated by ELISA for total IgG against JY1 peptide and we also analyzed the cross reactivity against a panel of 5 V3 peptides. The control group didn't generate detectable anti JY1 response, differently, the group immunized with the conjugate HBsAg-MAP8 (TT-JY1), generated strong responses versus the homologous peptide in the 100% of cases, it was also found a cross reactive response for more than 2 peptides from the panel, which increases with the number of inoculations. These results evidenced the critical role of conjugation for obtaining responses; the characteristic of antigen particulation improved the immune response against the coupled MAP. Furthermore, it was possible to demonstrate for the first time the strong immune response after mucosal immunization in sera and mucosal surfaces.

Other dendrimers with identical results were prepared according the examples 2 and 3 using as carrier molecules the core antigen from the Hepatitis B and C viruses and from Dengue Virus.

Furthermore, when using the peptide from the Gonadotropine Releasing Hormone as the synthetic part of the dendrimeric structure coupled to the already mentioned antigens, it was noted a significant reduction in the size of gonades respect to the control immunized with peptides alone and to the non-immunized mice.

### Example 13: Analysis of the cross neutralization of different HIV-1 isolates obtained using sera with high levels of cross reactivity.

With the aim of evaluating the cross neutralization of sera with high cross reactivity for the loop V3, an in vitro assay using different laboratory isolates from the HIV-1 was carried out. For these assays, sera pools were selected taking into consideration their level of cross reactivity. HBsAg-MAPS (TT-JY1) (example 4), HBsAg-MAP8 (TT-RF) (example 10) and the mixture HBsAg-MAP4 (CD4) with HBsAg-MAP4 (consensus) (example 8).

These studies showed that the antibody response induced by each immunogen have capacity to induce neutralizing effect heterologous isolates.

It was evidenced also that the mixture with CD4 increased the level of cross neutralization. Single peptides or their conjugate don't generates detectable cross neutralization.

### Example 14: Synthesis of dendrimeric structures employing different nucleus.

With the aim of increasing the number of B epitopes per molecular unit, an indirect synthesis is consisting in the synthesis of the nucleus and the peptides separately.

This system avoids the mistakes inherent to the synthesis of large sequences and enables a higher incorporation of B and T epitopes to the nucleus adequately functionalized.

The synthesis of the dendrimeric nucleus was carried out by reaction of the amino group of 1, 6 diaminohexano with the carboxyl groups, carboxyl of the amino acid Boc-Lys (Boc)-OH employing diciclohexilcabodiimide (DCC) as an activator. After that, the desprotection was made with trifluoroacetic acid (TFA) 37% in dichloromethane (DCM), obtaining a nucleus with 4 reactive ends, producing a tetravalent possibility of dendrimerization and a second and third levels of coupling for Boc-Lys (Boc)-OH, employing the same cycle of reactions for obtaining a nucleus with 16 amino reactive ends. After that, we make the reaction between those groups with the carboxyl group of the bromoacetic acid. The nucleus is purified by RP-HPLC employing a column C8.

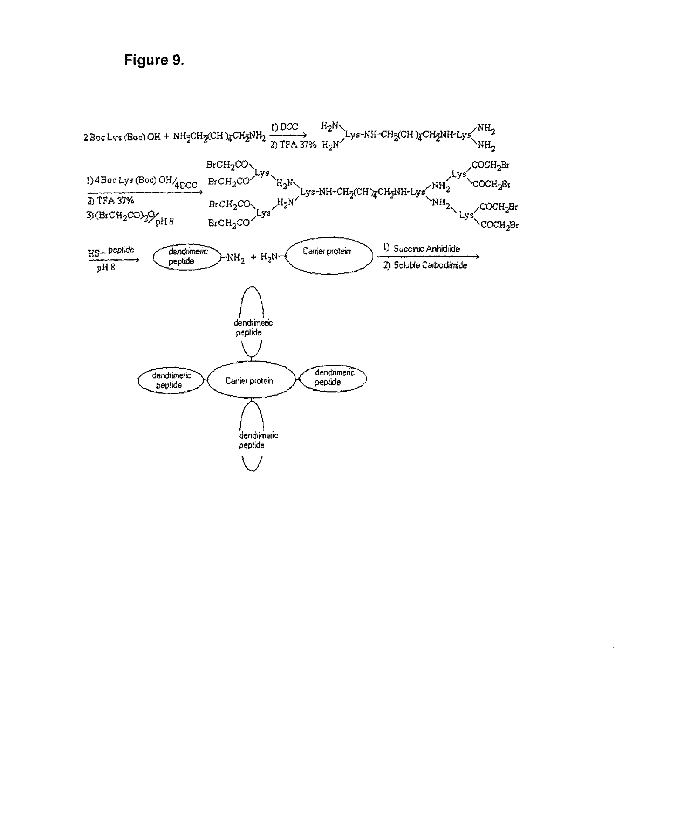

The JY1 peptide was synthesized previously containing a cystein in the C terminal end, which reacts in adequate proportions with the obtained nucleus. The tioether produced by the interaction between the hetero atom group from the nucleus and the tiol group from the cystein was accomplished in basic conditions (pH, 8-9) (fig.9). The degree of incorporation was determined by SDS-PAGE and gel filtration chromatography. Dendrimeric peptides were obtained between 40 and 50 kD.

The dendrimeric structure was coupled to different carrier proteins using the coupling agents already described in the example 2. The resulting dendrimeric structures composed by the protein and the MAPs were then used in different immunization schedules where it was evidenced their superiority in terms of immunogenicity and cross reactivity.

This system offered different advantages for example, various peptides B or T prepared separately can be incorporated inside a unique matrix adequately functionalized (4-6H), The antigenic peptides can be synthesized and conjugated through the C and N-ends as needed enabling the purification of individual determinants, and avoiding errors inherent to the synthesis of long sequences.

### Brief description of the drawings

### Figure 1.

A) Immunogenicity study after three and four doses. Groups: 1, MAP8 (TT-JY1) y 2, HBsAg-MAP8 (TT-JY1). Doses were 50 and 10 µg, respectively.
B) Study of cross-reacting Abs after three and four doses. Pooled sera from both groups were tested at a fixed dilution 100⁻¹. An indirect ELISA coating with V3-BSA conjugates was done.
C) Table 1. Amino acid sequences of V3 peptides from HIV-1tested.

### Figure 2.

Recognition study to several V3 peptides of pooled sera after four doses. Groups: 1, MAP8 (TT-JY1); 2, TAB9. Doses were 16,4 and 100 µg, respectively.

### Figure 3.

A) Table 2. Summary of the immunization schedule.
B) Comparative study of immunogenicity among different ways of presentation of JY1 epitope to the Immune System.

### Figure 4.

A) Table 3. Summary of the immunization schedule.
B) Recognition study to V3 peptides of sera elicited after inoculation of Ags presenting the JY1 epitope in different ways to the Immune System.

### Figure 5a.

Table 4. Summary of the immunization schedule.
Table 5. Amino acid sequences of CD4 binding peptide from gp120, consensus peptide from V3 and peptide library of V3 sequences from HIV-1.
Table 6. Immunogenicity results after four and five doses.

### Figure 5b.

Graphic 1. Study of cross-reacting Abs after five doses of sera from groups 2, 3 and 6 from table 3.

Graphic 2. Study of cross-reacting Abs after five doses of sera from groups 4, 5 y 6 from table 3.

### Figure 5c.

Table 7. Recognition study against a panel of V3 peptides synthesized on cellulose membrane. The quality profile shows the relative intensities of the recognition of pooled sera after four and five doses. 1 and 2, Mixture of conjugates HBsAg-MAP4 (peptide library of V3 sequences) and HBsAg-MAP4 (CD4 binding peptide from gp120); 3 and 4, Mixture of conjugates HBsAg-MAP4 (consensus peptide from V3) and HBsAg-MAP4 (CD4 binding peptide from gp120); 5, HBsAg after five doses; 6, HBsAg-MAP4 (CD4 binding peptide from gp120) after five doses. Four different color intensities show the quality of antibody interaction against peptides. White: no recognition, light gray: weak positive, dark gray: positive, black: strong positive.

### Figure 5d.

Pictures corresponding to results presented in table 5. Pictures: 1 and 2, Mixture of conjugates HBsAg-MAP4 (peptide library of V3 sequences) and HBsAg-MAP4 (CD4 binding peptide from gp120); 3 and 4, Mixture of conjugates HBsAg-MAP4 (consensus peptide from V3) and HBsAg-MAP4 (CD4 binding peptide from gp120; after four and five doses, respectively; 5, HBsAg after five doses; 6, HBsAg-MAP4 (CD4 binding peptide from gp120) after five doses. Pooled sera from all groups were tested at a fixed dilution 100⁻¹.

### Figure 6.

A) Table 8. Summary from the immunization schedule.
B) Determination of the immunogenicity of different groups after three doses.
C) Graphic 1. Study of the cross reactivity after three doses with the sera from groups 4-10 described in table 3. Pools of sera were assayed at 1/100 fixed dilution. The indirect ELISA was carried out covering the plates with the different peptides coupled to BSA.

Graphic 2. Study of the crossreactivity after fourth doses with sera from groups 4-10 described in table 3. Pools of sera were assayed at 1/100 fixed dilution. The indirect ELISA was carried out covering the plates with the different peptides coupled to BSA.

### Figure 7.

A) Study of recognition to various V3 peptides of sera pools after three doses. Group 1, MAP8 (TT-RF); 2, HBsAg-MAP8 (TT-RF). Doses were 40 y 10 µg respectively.

### Figure 8.

A) Table 8. Summary of the immunization schedule.
B) Immunogenicity evaluation by ELISA after three doses.

### Figure 9.

Obtaining of dendrimeric peptides and the antigenic structures.

## Claims

1. Method for obtaining antigenic structures able to enhance the specific cross reactivity comprising the steps:
A) selection of the epitope of interest;
B) obtaining of dendrimeric structures with the epitopes selected in (A), using a method of synthesis selected from the following group: 1) synthesis on a nucleus of aspartic acid or lysine; 2) synthesis on a nucleus of organic molecules and 3) direct coupling of peptides on a dendrimeric nucleus, the B and T-cell epitopes can be mixed on the same nucleus;
C) conjugation of one or various dendrimeric structures obtained in (B), to one or various carrier molecules using a method of coupling selected from the group consisting in: 1) conjugation using glutaraldehyde, 2) using succinic anhydride, 3) using the ester of the β-maleimidopropionic-N-hidroxisucci-nimidic acid (MPS), and 4) any method avoiding the use of spacer agents with aromatic side chains.
D) mixture of conjugates obtained in the step (C) including the addition of the CD4-binding peptide or a conjugate containing it; and
E) adjuvation of the resulting formulation in alum or a any other convenient adjuvant.

2. Method according to claim 1, wherein peptides contained in the structures obtained from step (B) belong to regions from the Human Immunodeficiency virus type 1 or 2.

3. Method according to claim 2, wherein peptides contained in the structures obtained from the step (B) belong to variable regions from the Human Immunodeficiency virus type 1 or 2.

4. Method according to claim 3, wherein peptides contained in the structures obtained from the step (B) belong to the third variable region (V3) from the Human Immunodeficiency Virus 1 or 2.

5. Method for according to claim 1, wherein peptides contained in the structures obtained from the step (B) belong to the Hepatitis B Virus.

6. Method according to claim 1, wherein peptides contained in the structures obtained from the step (B) belong to the Hepatitis C Virus.

7. Method according to claim 1, wherein peptides contained in the structures obtained from the step (B) belong to Dengue Virus.

8. Method according to claim 1, wherein the oligomers contained in the structures obtained from the step (B) belong to polysaccharidic regions from Neisseria meningitidis.

9. Method according to claim 1, wherein peptides contained in the structures obtained from the step (B) are mimetic peptides of antigenic polyssacharides.

10. Method according to claim 1, wherein peptides contained in the structures obtained from the step (B) belong to antigenic regions from a microorganism or a pathogen.

11. Method according to claim 1, wherein peptides contained in the structures obtained from the step (B) belong to self-antigens.

12. Method according to claim 11, wherein peptides contained in the structures obtained from the step (B) belong to the Gonadotropine Releasing Hormone.

13. Method according to claim 1, wherein the carrier molecule from the step (C) is the Hepatitis B surface antigen, HBsAg.

14. Method according to claim 1, wherein the carrier molecule from the step (C) is the p64K protein from Neisseria meningitidis.

15. Method according to claim 1, wherein the carrier molecule from the step (C) is the core protein from the Human Inmunodeficiency Virus.

16. Method according to claim 1, wherein the-carrier molecule from the step (C) is the hepatitis b core antigen or the Hepatitis C core antigen.

17. Method according to claim 1, wherein the carrier molecule from the step (C) is a polysaccharide.

18. Method according to claim 17, wherein the carrier molecule from the step (C) is a dextran.

19. Method according to claim 1, wherein the carrier molecule and the dendrimeric structure from the step (C) are coupled covalently, electrostatically or by hydrophobicity.

20. Antigenic structure able to enhance the specific cross reactivity comprising dendrimeric antigenic epitopes coupled to carrier proteins.

21. Antigenic structure according to claim 20 wherein the epitopes from the dendrimeric region have peptidic or oligosaccharidic nature.

22. Antigenic structure according to claim 21 wherein the epitopes of peptidic nature are peptidic consensus of variable regions selected from subtypes, subgroups, serotypes or other kind of associations.

23. Antigenic structure according to claim 21 wherein the epitopes of peptidic nature are peptidic libraries from peptidic sequences selected from microorganisms or mimetic peptides.

24. Antigenic structure according to claims 21 to 23 wherein the epitopes from the dendrimeric peptidic regions belong to the Human Immunodeficiency Virus 1 or 2.

25. Antigenic structure according to claims 24 wherein the epitopes from the dendrimeric peptidic regions belong to variable regions from the Human Immunodeficiency Virus 1 or 2.

26. Antigenic structure according to claim 25 wherein the epitopes from the dendrimeric peptidic regions belong to the V3 variable region from the Human Immunodeficiency Virus 1 or 2.

27. Antigenic structure according to claims 21 to 23 wherein the epitopes from the dendrimeric peptidic regions belong to regions from the Hepatitis B Virus.

28. Antigenic structure according to claims 21 to 23 wherein the epitopes from the dendrimeric peptidic regions belong to regions from the Hepatitis C Virus.

29. Antigenic structure according to claims 21 to 23 wherein the epitopes from the dendrimeric peptidic regions belong to regions from Dengue Virus.

30. Antigenic structure according to claim 21 wherein the epitopes from the dendrimeric peptidic regions belong to polysaccharidic antigens from Neisseria meningitidis.

31. Antigenic structure according to claims 21 to 23 wherein the epitopes from the dendrimeric peptidic regions are mimetic peptides from polysaccharidic or proteic antigens.

32. Antigenic structure according to claims 21 to 23 wherein the epitopes from the dendrimeric peptidic regions belong to microorganisms or pathogens.

33. Antigenic structure according to claims 21 to 23 wherein the epitopes from the dendrimeric peptidic regions belong to self-antigens.

34. Antigenic structure according to claims 21 to 23 wherein the epitopes from the dendrimeric peptidic regions belong to the Gonadotropine Releasing Hormone.

35. Antigenic structure according to claim 20 wherein the carrier molecule is the Hepatitis B Surface Antigen, HBsAg.

36. Antigenic structure according to claim 20 wherein the carrier molecule is the p64K protein from Neisseria meningitidis.

37. Antigenic structure according to claim 20 wherein the carrier molecule is the HIV nucleocapside antigen.

38. Antigenic structure according to claim 20 wherein the carrier molecule is the HBV nucleocapside antigen (HBcAg).

39. Antigenic structure according to claim 20 wherein the carrier molecule is a polysaccharide.

40. Antigenic structure according to claim 39 wherein the carrier molecule is a dextran.

41. Use of the antigenic structure according to claims 20 to 40 in a diagnostic system.

42. Use of the antigenic structure according to claims 20 to 40 in preventive and therapeutic vaccines.

43. Vaccine formulation comprising the antigenic structure of claims 20 to 40 adsorbed or adjuvated conveniently.

44. Vaccine formulation according to claim 43 for systemic or mucosal use.

45. Use of vaccine formulations of claims 43 and 44 to prevent infectious diseases, autoimmunity or cancer.

46. Use of vaccine formulations of claims 43 and 44 to treat infectious diseases, autoimmunity or cancer.
